# EUROPEAN PATENT APPLICATION

(11) **EP 3 335 730 A1**
(43) Date of publication of application: **20.06.2018**
(21) Application number: 16382602.7
(22) Date of filing: 15.12.2016
(51) Int. Cl.: A61K 45/06, A61K 31/436, A61K 31/137, A61K 31/19, A61K 31/198, A61K 31/355, A61K 31/385, A61K 31/397, A61K 31/4439, A61K 31/575, A61P 3/00, A61P 25/00, A61P 43/00

(54) **COMPOUNDS FOR TREATING X-LINKED ADRENOLEUKODYSTROPHY**

(71) Applicant: Fundació Institut de Recerca Biomédica de Bellvitge (IDIBELL), 08908 Barcelona (ES); Centro de Investigación Biomédica en Red (CIBER), 28029 Madrid (ES)
(72) Inventor: PUJOL ONOFRE, Aurora, E-08015 Barcelona (ES)
(74) Representative: ABG Patentes, S.L.

(57) **Abstract**

The invention relates to a compound of formula (I) 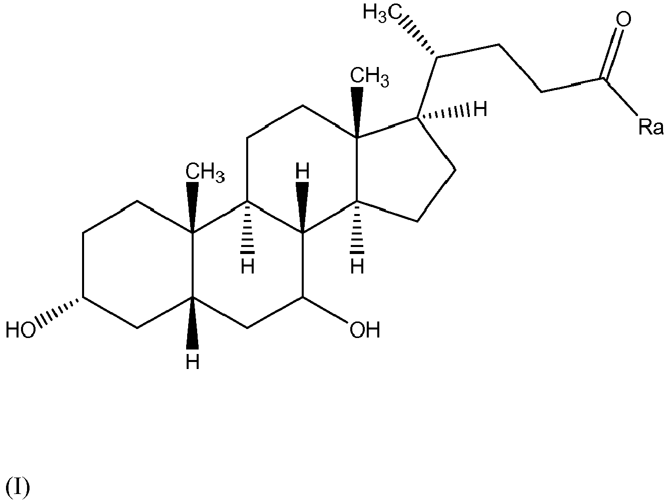 as described in the invention, or a pharmaceutically acceptable salt, stereoisomer or solvate thereof, or a mixture thereof for use in the prevention and/or treatment of a disease caused by loss of function of ABCD1 gene, a disease caused by accumulation of very long fatty acids in organs and plasma or of a peroxisomal disorder. The invention also relates to combinations comprising (i) a compound of formula (I) and ii) an antioxidant, an activator of mitochondria biogenesis, an antiinflammatory compound, an activator of autophagy, a peroxisomal biogenesis activator or a mixture thereof, and the medical use of said combinations.

## Description

### TECHNICAL FIELD OF THE INVENTION

The invention is related to the field of medical treatments of a disease caused by loss of function of ABCD1 gene, a disease caused by accumulation of very long fatty acids in organs and plasma or of a peroxisomal disorder.

### BACKGROUND OF THE INVENTION

X-linked adrenoleukodystrophy (X-ALD: McKusick no. 300100) is a rare, fatal neurometabolic disease characterized by central inflammatory demyelination in the brain or slowly progressive spastic paraparesis resulting from axonal degeneration in the spinal cord. All patients with X-linked adrenoleukodystrophy have mutations in the ABCD1 (ALD) gene (Xq28) that encodes for the peroxisomal adrenoleukodystrophy protein (ALDP), which in turn transports very long chain fatty acids (VLCFA) or VLCFA-CoA esters into the peroxisome to be degraded by β-oxidation. The two main neurological phenotypes are the severe childhood cerebral form (cCALD), which progresses rapidly and is associated with an inflammatory response in the brain white matter, and the slowly progressing adult adrenomyeloneuropathy (AMN), which presents distal axonopathy in spinal cord and peripheral neuropathy. About 35% of AMN patients develop cerebral demyelination (cAMN); they share the same poor prognosis as children with cerebral ALD.

Therapeutic options are scarce; for the cerebral forms of the disease, when caught very early, bone marrow transplant or, recently, hematopoietic stem cell gene therapy approaches may be efficacious Biffi A . et al., Hum Mol Genet 2011; 20:R42-53, Cartier N. et al., Brain Pathol 2010; 20:857-62.-. No pharmacological treatment has proved beneficial as yet.

Therefore, there is still a need in the state of the art to identify suitable and effectives compounds for the prevention and /or treatment of diseases caused by loos of function of ABCD1 gene, disease caused by accumulation of very long fatty acids or peroxisomal disorders.

### SUMMARY OF THE INVENTION

In a first aspect, the invention relates to a compound of formula I 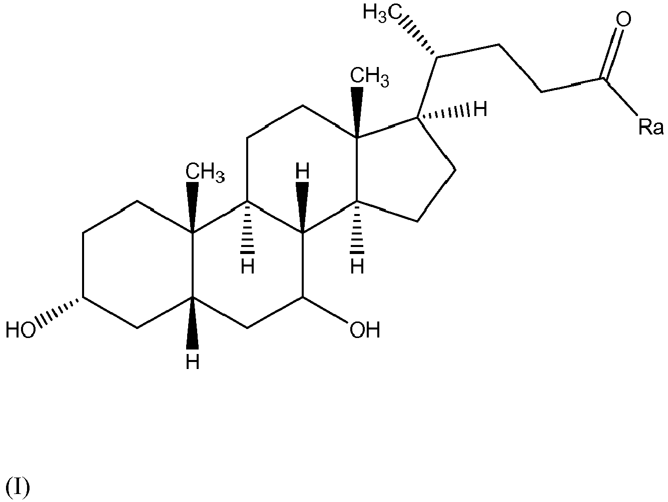 wherein Ra is -NR-CH-R₁-R₂ or -OH, wherein R is -H or C₁-C₄ alkyl; R₁ is -CH₂-SO₃R₃ and R₂ is -H; or R₁ is -COOH and R₂ is -CH₂-CH₂-CONH₂, -CH₂-CONH₂, -CH₂-CH₂-SCH₃ or -CH₂-S-CH₂-COOH; and R₃ is -H or the radical of a basic amino acid, or a pharmaceutically acceptable salt, stereoisomer, solvate or prodrug thereof, or a mixture thereof for use in the prevention and/or treatment of a disease caused by loss of function of ABCD1 gene, a disease caused by accumulation of very long fatty acids in organs and plasma or of a peroxisomal disorder.

In a second aspect, the invention relates to a combination comprising (i) a compound of formula (I) 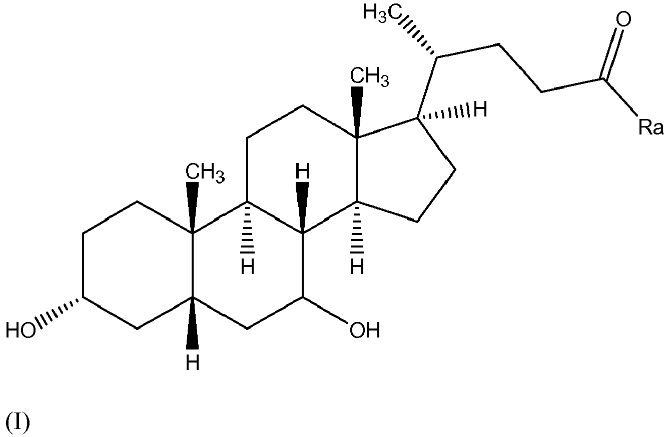 wherein Ra is -NR-CH-R₁-R₂ or -OH, wherein R is -H or C₁-C₄ alkyl; R₁ is -CH₂-SO₃R₃ and R₂ is -H; or R₁ is -COOH and R₂ is -CH₂-CH₂-CONH₂, -CH₂-CONH₂, -CH₂-CH₂-SCH₃ or -CH₂-S-CH₂-COOH; and R₃ is -H or the radical of a basic amino acid, or a pharmaceutically acceptable salt, stereoisomer, solvate or prodrug thereof, or a mixture thereof and (ii) an antioxidant, an activator of mitochondria biogenesis, an anti-inflammatory compound, an activator of autophagy, a peroxisome biogenesis activator or a mixture thereof.

In a third aspect, the invention relates to the compound of the invention or the combination of the invention for use the prevention and/or treatment of a disease caused by loss of function of ABCD1 gene, a disease caused by accumulation of very long fatty acids in organs and plasma or of a peroxisomal disorder.

### BRIEF DESCRIPTION OF THE DRAWINGS

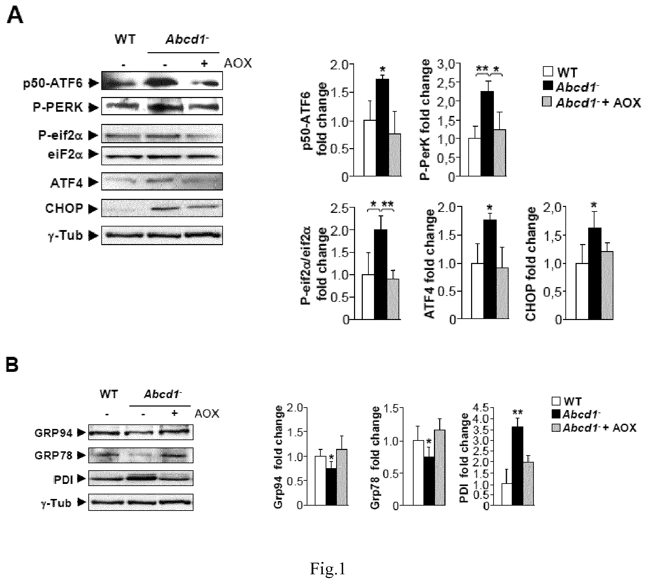

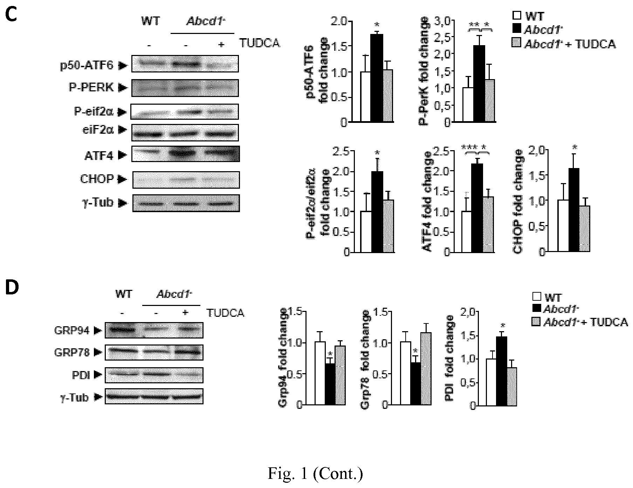

**Figure 1****. Antioxidant and TUDCA treatments prevent ER stress in the X-ALD mouse model.**
   A, C) Antioxidants (AOX) (A) and TUDCA (C) prevent activation of ATF6, PERK and eif2α phosphorylation (P-eif2α), and induction of ATF4 and CHOP. B, D) Antioxidants (AOX) (B) and TUDCA (D) normalized the ER chaperones GRP94 and GRP78 and also the PDI levels in Abcd1- mouse spinal cords.
   For all experiments γ-tubulin (γTub) was used as loading control (mean ± SD, n=8 per genotype; the right panels stand for the ratios of respective proteins relative to wild type (WT) littermates. Significant differences were determined as described in Materials and Methods (*p < 0.05, **p < 0.01).

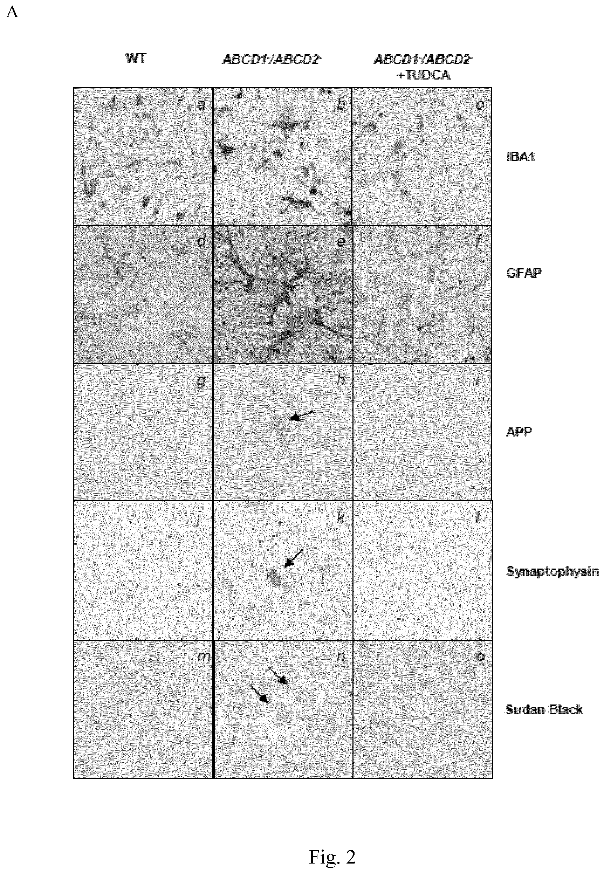

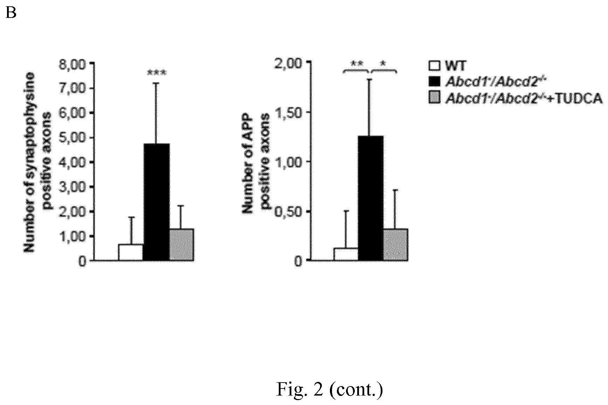

**Figure 2****. TUDCA halts axonal degeneration in** ***Abcd1**⁻***/*****Abcd2***^{*-*/*-*} **mice.**
   **A)** Immunohistological analysis of axonal damages performed in 17-month-old WT, *Abcd1⁻*/*Abcd2*^{*-*/*-*} and *Abcd1⁻*/*Abcd2*^{*-*/*-*} mice treated with TUDCA (*Abcd1 ⁻*/*Abcd2*^{*-*/*-*} + TUDCA). Spinal cord immunohistological sections were processed for **a**-**c** Iba-1, **d-f** GFAP, **g**-**i** synaptophysin, **j**-l APP and **m**-**o** Sudan black. Representative images **a, d, g, j** and **m** for WT, b, e, **h, k,** and **n** for *Abcd1⁻* /*Abcd2*^{*-*/*-*}, and **c, f, i, l** and o for *Abcd1⁻* /*Abcd2*^{*-*/*-*} + TUDCA mice are shown. Bars 25 µm.
   **B)** Quantification of synaptophysin and APP accumulation in spinal cord immunohistological sections of WT, *Abcd1⁻*/*Abcd2*^{*-*/*-*} and *Abcd1 ⁻*/*Abcd2*^{*-*/*-*} + TUDCA mice (mean ± SD, n=6 per genotype).

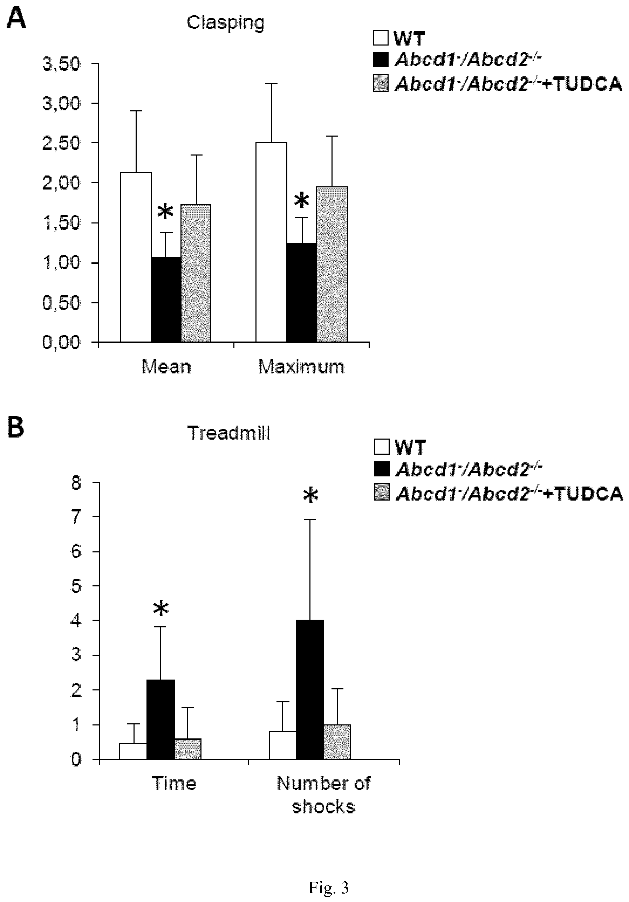

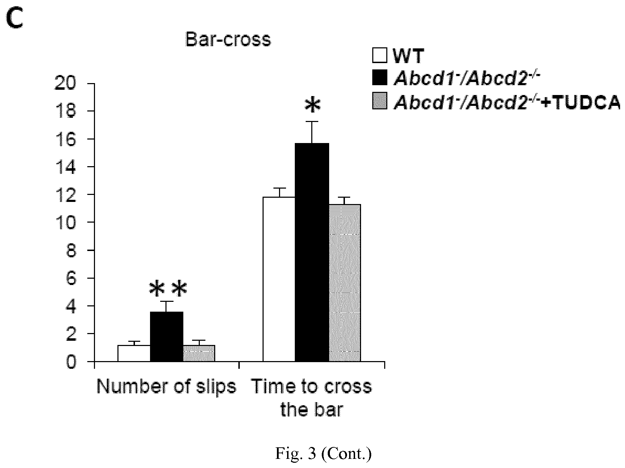

**Figure 3** **.TUDCA prevents locomotor deficit in *Abcd1⁻*/*Abcd2*^{*-*/*-*} mice.**
   Clasping **(A)**, treadmill **(B)** and bar cross **(C)** tests have been carried out in 17-month-old WT *Abcd*1*⁻*/*Abcd2* ^{*-*/*-*} and *Abcd1⁻*/*Abcd2*^{*-*/*-*} mice treated with TUDCA (*Abcd*1*⁻*/*Abcd2*^{*-*/*-*} + TUDCA). **A)** Hindlimb reflex was analysed for 10 s in three consecutive trials separated by 5 min rest. The best performance score/The average of the three trials of each animal was taken for statistical analysis (Dumser M. et al., Acta Neuropathol 2007; 114:573-86). **B**) The latency to falling off the belt (time of shocks) and the number of shocks received were computed after 5 min. **C)** The time spent to cross the bar and the numbers of slips were quantified. For all experiments, values are expressed as mean ± SD (mean ± SD, n=6 per genotype) and significant differences were determined as described in Materials and Methods (*p < 0.05, **p < 0.01).

### DETAILED DESCRIPTION OF THE INVENTION

The authors of the invention found that the chemical chaperone tauroursodeoxycholic acid (TUDCA), prevented ER stress in spinal cords, together with halting the axonopathy and the associated locomotor impairment in the X-ALD mouse model (Examples 3-4).

### Combination of the invention

In an aspect, the invention relates to a combination comprising (i) a compound of formula (I) 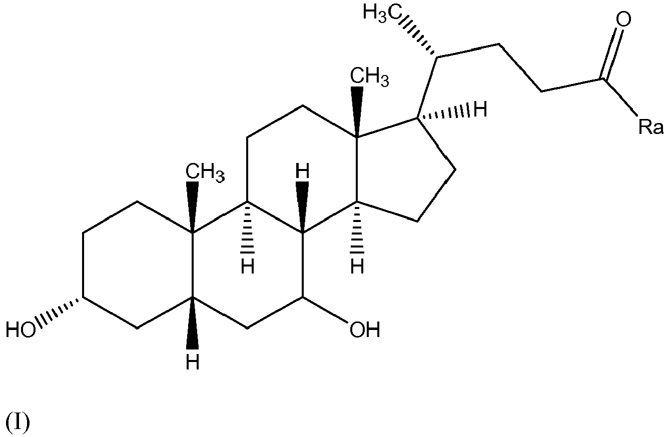 wherein Ra is -NR-CH-R₁-R₂ or -OH, wherein R is -H or C₁-C₄ alkyl; R₁ is -CH₂-SO₃R₃ and R₂ is -H; or R₁ is -COOH and R₂ is -CH₂-CH₂-CONH₂, -CH₂-CONH₂, -CH₂-CH₂-SCH₃ or -CH₂-S-CH₂-COOH; and R₃ is -H or the radical of a basic amino acid, or a pharmaceutically acceptable salt, stereoisomer, solvate or prodrug thereof, or a mixture thereof and (ii) an antioxidant, an activator of mitochondria biogenesis, an anti-inflammatory compound, an activator of autophagy, a peroxisome biogenesis activator or a mixture thereof.

"C1-C4 alkyl", as used herein, refers to a straight or branched hydrocarbon chain radical consisting of 1 to 4 carbon and hydrogen atoms, containing no unsaturation, and which is attached to the rest of the molecule by a single bond. Methyl, ethyl, n-propyl, iso-propyl and butyl, including n-butyl, tert-butyl, sec-butyl and iso-butyl are particularly preferred alkyl groups. As used herein, the term alkyl, unless otherwise stated, refers to both cyclic and noncyclic groups, although cyclic groups will comprise at least three carbon ring members, such as cyclopropyl or cyclohexyl. Alkyl radicals may be optionally substituted by one or more substituents, such as an aryl group, like in benzyl or phenethyl.

"Radical of a basic amino acid" relates to a residue or functional group of an amino acid that has basic side chains at neutral pH, particularly arginine, lysine, ornithine and histidine and having unpaired valance electrons. Said residues contain nitrogen and resemble ammonia, which is a base.

The term "salt" is to be understood as meaning any form of the active compound according to the invention in which this assumes an ionic form or is charged and is coupled with a counter-ion (a cation or anion) or is in solution. By this are also to be understood complexes of the active compound with other molecules and ions, in particular complexes which are complexed via ionic interactions. The definition includes in particular physiologically acceptable salts; this term must be understood as equivalent to "pharmacologically acceptable salts" or "pharmaceutically acceptable salts".

The term "physiologically acceptable salt" or "pharmaceutically acceptable salt" is understood in particular, in the context of this invention, as a salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals.

For instance, pharmaceutically acceptable salts of compounds provided herein are synthesized from the parent compound, which contains a basic or acidic moiety, by conventional chemical methods. Generally, such salts are, for example, prepared by reacting the free acid or base forms of these compounds with a stoichiometric amount of the appropriate base or acid in water or in an organic solvent or in a mixture of both. Generally, non-aqueous media like ether, ethyl acetate, ethanol, 2-propanol or acetonitrile are preferred. Examples of the acid addition salts include mineral acid addition salts such as, for example, hydrochloride, hydrobromide, hydroiodide, sulfate, nitrate, phosphate, and organic acid addition salts such as, for example, acetate, trifluoroacetate, maleate, fumarate, citrate, oxalate, succinate, tartrate, malate, mandelate, methanesulfonate and p-toluenesulfonate. Examples of the alkali addition salts include inorganic salts such as, for example, sodium, potassium, calcium and ammonium salts, and organic alkali salts such as, for example, ethylenediamine, ethanolamine, N,N-dialkylenethanolamine, triethanolamine and basic aminoacids salts. Since hydroxytyrosol has three hydroxyl groups, alkali addition salts are particularly preferred such as Na+ and NX4+ (wherein X is independently selected from H or a C1-C4 alkyl group).

For those persons skilled in the art, it will be evident that the scope of the present invention also includes salts which are not pharmaceutically acceptable as possible means for obtaining pharmaceutically acceptable salts.

Any compound referred to herein is intended to represent such specific compound as well as certain variations or forms. The compounds of the present invention represented by the above described formula (I) include stereoisomers. The term "stereoisomer" as used herein includes any enantiomer, diastereomer or geometric isomer (E/Z) of such compound. In particular, compounds referred to herein may have asymmetric centres and therefore exist in different enantiomeric or diastereomeric forms. Thus any given compound referred to herein is intended to represent any one of a racemate, one or more enantiomeric forms, one or more diastereomeric forms, and mixtures thereof. Likewise, stereoisomerism or geometric isomerism related to a double bond is also possible, therefore in some cases the molecule could exist as (E)-isomer or (Z)-isomer (trans and cis isomers). If the molecule contains several double bonds, each double bond will have its own stereoisomerism, that could be the same or different than the stereoisomerism of the other double bonds of the molecule. All the stereoisomers including enantiomers, diastereoisomers and geometric isomers of the compounds referred to herein, and mixtures thereof, are considered within the scope of the present invention.

The compounds of the invention may be in crystalline form either as free compounds or as solvates (e.g. hydrates, alcoholates, particularly methanolates) and it is intended that both forms are within the scope of the present invention. Solvate may include water or non-aqueous solvents such as ethanol, isopropanol, DMSO, acetic acid, ethanolamine, and EtOAc. Solvates, wherein water is the solvent molecule incorporated into the crystal lattice, are typically referred to as "hydrates". Hydrates include stoichiometric hydrates as well as compositions containing variable amounts of water. Methods of solvation are generally known within the art.

When a disclosed compound is named or depicted by structure, it is to be understood that the compound, including solvates thereof, may exist in crystalline forms, non-crystalline forms or a mixture thereof. The compounds or solvates may also exhibit polymorphism (i.e. the capacity to occur in different crystalline forms). These different crystalline forms are typically known as "polymorphs". It is to be understood that when named or depicted by structure, the disclosed compounds and solvates (e.g., hydrates) also include all polymorphs thereof. Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. One of ordinary skill in the art will appreciate that different polymorphs may be produced, for example, by changing or adjusting the conditions used in solidifying the compound. For example, changes in temperature, pressure, or solvent may result in different polymorphs. In addition, one polymorph may spontaneously convert to another polymorph under certain conditions.

Furthermore, any compound referred to herein may exist as tautomers. Specifically, the term tautomer refers to one of two or more structural isomers of a compound that exist in equilibrium and are readily converted from one isomeric form to another. Common tautomeric pairs are enamine-imine, amide-imidic acid, keto-enol, lactam-lactim, etc.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by ¹³C- or ¹⁴C-enriched carbon, or the replacement of at least one nitrogen by ¹⁵N-enriched nitrogen are within the scope of this invention.

The invention also provides "prodrugs" of the compounds described in the present description. The term "prodrug", as used herein, is intended to represent covalently bonded carriers, which are capable of releasing the compound of formula (I) as active ingredient when the prodrug is administered to a mammalian subject. Release of the active ingredient occurs *in vivo.* Prodrugs can be prepared by techniques known to one skilled in the art. These techniques generally modify appropriate functional groups in a given compound. These modified functional groups however regenerate original functional groups by routine manipulation or *in vivo.* Prodrugs of compounds of the invention include compounds wherein a hydroxy, amino, carboxylic, or a similar group is modified. Examples of prodrugs include, but are not limited to esters (e.g., acetate, formate, and benzoate derivatives), carbamates (e.g., N,N-dimethylaminocarbonyl) of hydroxy or amino functional groups in compounds of Formula (I)), amides (e.g., trifluoroacetylamino, acetylamino, and the like), and the like.

The invention also provides "metabolites" of the compounds described in the present description. A "metabolite" of a compound disclosed herein is a derivative of that compound that is formed when the compound is metabolized. The term "active metabolite" refers to a biologically active derivative of a compound that is formed when the compound is metabolized. The term "metabolized," as used herein, refers to the sum of the processes (including, but not limited to, hydrolysis reactions and reactions catalyzed by enzymes) by which a particular substance is changed by an organism. Thus, enzymes may produce specific structural alterations to a compound. For example, cytochrome P450 catalyzes a variety of oxidative and reductive reactions while uridine diphosphate glucuronyltransferases catalyze the transfer of an activated glucuronic-acid molecule to aromatic alcohols, aliphatic alcohols, carboxylic acids, amines and free sulphydryl groups. In a preferred embodiment, the metabolite accumulates in the brain or in the spinal cord.

The compounds of formula (I) or their salts, stereoisomers, solvates or prodrugs are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its pharmaceutically acceptable salt, stereoisomer or solvate.

In additional preferred embodiments, the preferences described above for the different groups and substituents in the formulae above are combined. The present invention is also directed to such combinations.

In a preferred embodiment, the compound of formula (I) is 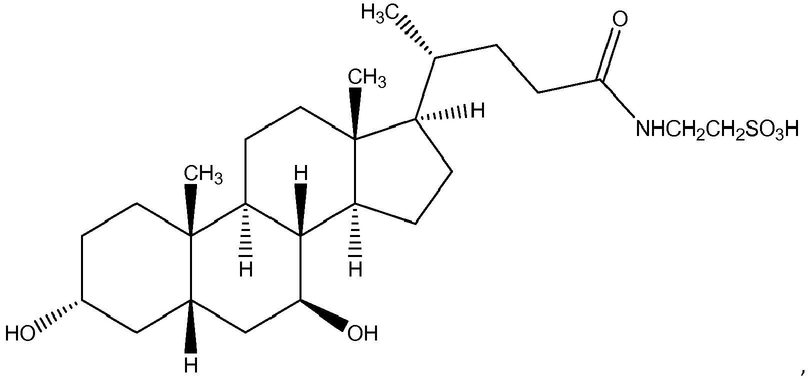 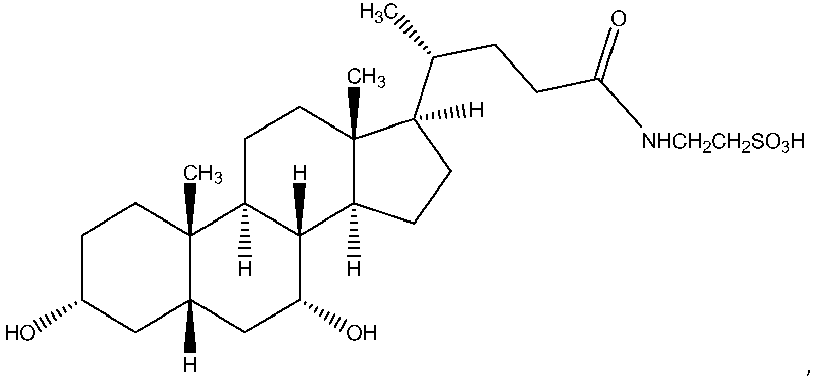 or

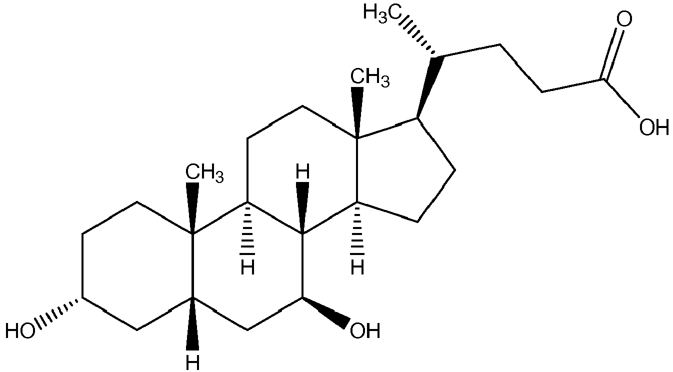

"TUDCA" relates to 3α,7β-Dihydroxy-5β-cholanoyltaurine, also known as UR 906; Ursodeoxycholyltaurine or Tauroursodeoxycholic acid, relates to an ambiphilic bile acid having CAS number 14605-22-2. It is the taurine conjugate form of ursodeoxycholic acid (UDCA).

"Taurochenodeoxycholic acid" relates to -[4-[(3*R*,5*S*,7*R*,8*R*,9*S*,10*S*,13*R*,14*S*,17*R*)-3,7-dihydroxy-10,13-dimethyl-2,3,4,5,6,7,8,9,11,12,14,15,16,17-tetradecahydro-1*H*-cyclopenta[*a*]phenanthren-17-yl]pentanoylamino]ethanesulfonic acid also known as 12-Deoxycholyltaurine; 12-Desoxycholyltaurine; Chenodeoxycholyltaurine or Chenyltaurine relates to a bile acid having a CAS number 6009-98-9.

"Ursodeoxycholic acid" relates to 3α,7β-dihydroxy-5β-cholan-24-oic acid or (R)-4-((3R,5S,7S,8R,9S,10S,13R,14S,17R)-3,7-dihydroxy- 10,13-dimethylhexadecahydro- 1H-cyclopenta[a]phenanthren-17-yl)pentanoic acid also known as ursodiol, having a CAS number 128-13-2, is one of the secondary bile acids which are metabolic byproducts of intestinal bacteria.

The combination of the invention may comprise a mixture of compounds (i). In addition, the combination of the invention comprises (ii) an antioxidant, an activator of mitochondria biogenesis, an anti-inflammatory compound, an activator of autophagy, a peroxisome biogenesis activator or a mixture thereof.

"Antioxidant", as used herein relates to a molecule that inhibits the oxidation of other molecules. Antioxidants can act by scavenging biologically important reactive free radicals or other reactive oxygen species (.O₂ -, H₂O₂, .OH, HOCl, ferryl, peroxyl, peroxynitrite, and alkoxyl), or by preventing their formation, or by catalytically converting the free radical or other reactive oxygen species to a less reactive species. Relative antioxidant activity can be measured by cyclic voltametry studies. The voltage at which the first peak occurs is an indication of the voltage at which an electron is donated, which in turn is an index of antioxidant activity. Alternatively,the antioxidant activity of a compound may be assayed by Fenton reaction.

The antioxidant according to the invention is selected from the group consisting of probucol, vitamin C and E, coenzyme Q- 10, glutathione, L-cysteine, N-acetylcysteine, lipoic acid, uric acid, carotenes, ubiquinol and α-tocoferol.

In a more preferred embodiment, the antioxidant is selected from the group consisting of vitamin E, α-lipoic acid, N-acetyl-cysteine and combinations thereof. In another preferred embodiment the combination of the invention comprises more than one antioxidant. In an even more preferred embodiment, the combination of the invention comprises vitamin E, α-lipoic acidic acid and N-acetyl-cysteine.

"Vitamin E" as used herein refers to all tocopherols, i.e. α-, β- and γ-tocopherol in all steric forms, as well as to physiologically acceptable esters thereof such as the acetates.

"α-lipoic acid" also known as lipoic acid (LA), α-lipoic acid, alpha lipoic acid (ALA) and thioctic acid is an organosulfur compound derived from octanoic acid having the CAS number 1077-28-7.

"N-acetyl-cysteine" also known as acetylcysteine, or N-acetyl-L-cysteine (NAC) is a prodrug for L-cysteine having the CAS number 616-91-1.

"Activator of mitochondria biogenesis", as used herein relates to a compound capable of increasing the individual mitochondrial mass and copy number. PGC-1α, a member of the peroxisome proliferator-activated receptor gamma (PGC) family of transcriptional coactivators, is the master regulator of mitochondrial biogenesis, therefore, activators of PGC-1α or activators of the PGC-1α signaling pathway such as activators of SIRT1, particularly resveratrol, or activator of AMP, such as ICAR, or activators of the PGC-1α signaling pathway such as bezafibrate may be used in the present invention. In addition, thiazolidinediones including pioglitazone regulates the expression of genes involved in mitochondrial biogenesis. Suitable methods for assaying if a compound is an activator of mitochondria biogenesis are known by a skilled person, for example an assay for quantitatively measure the levels of mitochondrial proteins whose presence depends on mitochondrial replication and protein synthesis, or a respirometric assay.

In a preferred embodiment, the activator of mitochondria biogenesis is pioglitazone.

"Pioglitazone", as used herein relates to (RS)-5-(4-[2-(5-ethylpyridin-2-yl)ethoxy]benzyl)thiazolidine-2,4-dione having a CAS number 111025-46-8.

"Anti-inflammatory compound" is understood as a chemical or biological substance which inhibits or reduces inflammation. Anti-inflammatory agents which can be used in the present invention include but are not limited to salicylates, such as acetylsalicylic acid, diflunisal and salsalate; propionic acid derivatives, such as ibuprofen, naproxen, fenoprofen, ketoprofen, dexketoprofen, flurbiprofen, oxaprozin, loxoprofen; acetic acid derivatives, such as indomethacin, sulindac, etodolac, ketorolac, diclofenac, nabumetone; enolic acid derivatives, such as piroxicam, meloxicam, tenoxicam, droxicam, lornoxicam and isoxicam; fenamic acid derivatives such as mefenamic acid, meclofenamic acid, flufenamic acid, and tolfenamic acid; selective COX-2 inhibitors such as celecoxib, rofecoxib, valdecoxib, parecoxib, lumiracoxib, etoricoxib and firocoxib; sulphonanilides such as nimesulide; and other compounds such as licofelone.

In a preferred embodiment, the anti-inflammatory compound is fingolimod or siponimod.

"Fingolimod" as used herein relates to 2-amino-2-[2-(4-octylphenyl)ethyl]propane-1,3-diol, a sphingosine-1-phosphate receptor modulator an immunomodulating drug with CAS number 162359-55-9.

"Siponimod",or BAF312 as used herein, relates to 1-{4-[(1E)-N-{[4-cyclohexyl-3-(trifluoromethyl)benzyl]oxy}ethanimidoyl]-2-ethylbenzyl}-3-azetidinecarboxylic acid, a selective sphingosine-1-phosphate receptor modulator having a CAS number 1230487-00-9.

"Activator of autophagy", as used herein relates to a compound that induces a catabolic cell mechanism that involves cell degradation of unnecessary or dysfunctional cellular components such as intracellular proteins, protein aggregates, cellular organelles, cell membranes, organelle membranes and other cellular components through the actions of lysosomes. While it is closely linked with apoptosis, autophagy is primarily characterized as a catabolic mechanism by which cellular energy homeostasis is maintained. Suitable activators of autophagy are inhibitors of mTOR, inositol 1,4,5-trisphosphate (IP3), epidermal growth factor receptor (EGFR), Bcl-2, and Bcl-xl.

In a preferred embodiment, the activator of autophagy is temsirolimus. "Temsirolimus", as used herein relates to 1R,2R,4S)-4-{(2R)-2-[(3S,6R,7E,9R,10R,12R,14S,15E,17E,19E,21S,23S,26R,27R,34aS)-9,27-dihydroxy-10,21-dimethoxy-6, 8,12,14,20,26-hexamethyl-1,5,11,28,29-pentaoxo-1,4,5,6,9,10,11,12,13,14,21,22,23,24,25,26,27,28,29,31,32,33,34,34a-tetracosahydro-3H-23,27-epoxypyrido[2,1-c][[1,4]oxazacyclohentriacontin-3-yl]propyl}-2-methoxycyclohexyl 3-hydroxy-2-(hydroxymethyl)-2-methylpropanoate, a specific mTOR inhibitor, having a CAS number 162635-04-3.

"A peroxisome biogenesis activator", as used herein relates to a compound capable of increasing the individual peroxisomal copy number directly or by inducing the expression of genes involved in peroxisomes formation.

In a preferred embodiment, the peroxisome biogenesis activator is sodium phenylbutirate or 4-phenylbutyrate.

Sodium Phenylbutirate, as uses herein relates to a salt of an aromatic fatty acid, 4-phenylbutyrate (4-PBA) or 4-phenylbutyric acid having the CAS number 1716-12-7.

In a preferred embodiment, the combination of the invention comprises in addition to the element (i) two additional compounds selected from the group consisting of an antioxidant, an activator of mitochondria biogenesis, an anti-inflammatory compound, an activator of autophagy, a peroxisome biogenesis activator and a mixture thereof. Thus, the combination of the invention comprise in addition to the element (i) an antioxidant and an activator of mitochondria biogenesis; an antioxidant and an anti-inflammatory compound; an antioxidant and an activator of autophagy; an activator of mitochondria biogenesis and an antioxidant; an activator of mitochondria biogenesis and an anti-inflammatory compound; an activator of mitochondria biogenesis and an activator of autophagy; an anti-inflammatory compound and an antioxidant; an anti-inflammatory compound and an activator of mitochondria biogenesis; an anti-inflammatory compound and an activator of autophagy; an activator of autophagy and an antioxidant; an activator of autophagy and an activator of mitochondria biogenesis; an activator of autophagy and an anti-inflammatory compound, an antioxidant and a peroxisome biogenesis activator, an activator of mitochondria biogenesis and a peroxisome biogenesis activator, an anti-inflammatory compound and a peroxisome biogenesis activator or an activator of autophagy and a peroxisome biogenesis activator.

In another preferred embodiment, the combination of the invention comprises in addition to the element (i) three additional compounds selected from the group consisting of an antioxidant, an activator of mitochondria biogenesis, an anti-inflammatory compound, an activator of autophagy, a peroxisome biogenesis activator and a mixture thereof

Thus, the combination of the invention comprise in addition to the element (i) an antioxidant, an activator of mitochondria biogenesis and an anti-inflammatory compound; an antioxidant, an activator of mitochondria biogenesis and an activator of autophagy; an antioxidant, an anti-inflammatory compound and an activator of mitochondria biogenesis; an antioxidant, an anti-inflammatory compound and an activator of autophagy; an antioxidant, an activator of autophagy and an activator of mitochondria biogenesis; an antioxidant, an activator of autophagy and an anti-inflammatory compound; an activator of mitochondria biogenesis, an antioxidant and an anti-inflammatory compound; an activator of mitochondria biogenesis, an antioxidant and an activator of autophagy; an activator of mitochondria biogenesis, an anti-inflammatory compound and an antioxidant; an activator of mitochondria biogenesis, an anti-inflammatory compound and an activator of autophagy; an activator of mitochondria biogenesis, an activator of autophagy and an antioxidant; an activator of mitochondria biogenesis, an activator of autophagy and an anti-inflammatory compound; an anti-inflammatory compound, an antioxidant and an activator of mitochondria biogenesis; an anti-inflammatory compound, an antioxidant and an activator of autophagy; an anti-inflammatory compound, an activator of mitochondria biogenesis and an antioxidant; an anti-inflammatory compound, an activator of mitochondria biogenesis and an activator of autophagy; an anti-inflammatory compound, an activator of autophagy and an antioxidant; an anti-inflammatory compound, an activator of autophagy and an activator of mitochondria biogenesis; an activator of autophagy, an antioxidant and an activator of mitochondria biogenesis; an activator of autophagy, an antioxidant and an anti-inflammatory compound; an activator of autophagy, an activator of mitochondria biogenesis and an antioxidant; an activator of autophagy, an activator of mitochondria biogenesis and an anti-inflammatory compound; an activator of autophagy, an anti-inflammatory compound and an antioxidant; or an activator of autophagy, an anti-inflammatory compound and an activator of mitochondria biogenesis, an antioxidant, an activator of mitochondria biogenesis and a peroxisome biogenesis activator; an antioxidant, an anti-inflammatory compound and a peroxisome biogenesis activator; an antioxidant, an activator of autophagy and a peroxisome biogenesis activator; an activator of mitochondria biogenesis, an antioxidant and a peroxisome biogenesis activator; an activator of mitochondria biogenesis, an anti-inflammatory compound and a peroxisome biogenesis activator; an activator of mitochondria biogenesis, an activator of autophagy and a peroxisome biogenesis activator; an anti-inflammatory compound, an antioxidant and a peroxisome biogenesis activator; an anti-inflammatory compound, an activator of mitochondria biogenesis and a peroxisome biogenesis activator; an anti-inflammatory compound, an activator of autophagy and a peroxisome biogenesis activator; an activator of autophagy, an antioxidant and a peroxisome biogenesis activator; an activator of autophagy, an activator of mitochondria biogenesis and a peroxisome biogenesis activator; an activator of autophagy, an anti-inflammatory compound and a peroxisome biogenesis activator.

In another preferred embodiment, the combination of the invention comprises in addition to the element (i) four additional compounds selected from the group consisting of an antioxidant, an activator of mitochondria biogenesis, an anti-inflammatory compound, an activator of autophagy, a peroxisome biogenesis activator and a mixture thereof Thus, the combination of the invention comprise in addition to the element (i) and an antioxidant, an activator of mitochondria biogenesis, an anti-inflammatory compound and an activator of autophagy; an antioxidant, an activator of mitochondria biogenesis, an anti-inflammatory compound and a peroxisome biogenesis activator; an antioxidant, an activator of mitochondria biogenesis, an activator of autophagy and a peroxisome biogenesis activator; an antioxidant, an anti-inflammatory compound, an activator of autophagy and a peroxisome biogenesis activator; an activator of mitochondria biogenesis, an anti-inflammatory compound, an activator of autophagy and a peroxisome biogenesis activator. In another preferred embodiment of the combination of the invention comprises in addition to the element (i) an antioxidant, an activator of mitochondria biogenesis, an anti-inflammatory compound, a peroxisome biogenesis activator and an activator of autophagy.

A combination of the invention may be formulated for its simultaneous, separate or sequential administration. This has the implication that the combination of the at least two compounds may be administered:
- as a combination that is being part of the same medicament formulation, the at least two compounds being then administered always simultaneously (i.e. forming part of the same pharmaceutical composition).
- as a combination of two units, each with one of the substances giving rise to the possibility of simultaneous, sequential or separate administration.

In a particular embodiment, the compound (i) is independently administered from the antioxidant, an activator of mitochondria biogenesis, an anti-inflammatory compound, an activator of autophagy, a peroxisomal biogenesis activator or a mixture thereof (i.e in two units) but at the same time.

In another particular embodiment, the compound (i) is administered first, and then the antioxidant, the activator of mitochondria biogenesis, the anti-inflammatory compound, the activator of autophagy, a peroxisomal biogenesis activator or a mixture thereof is separately or sequentially administered.

### Medical uses

In another aspect, the invention relates to a compound of formula I 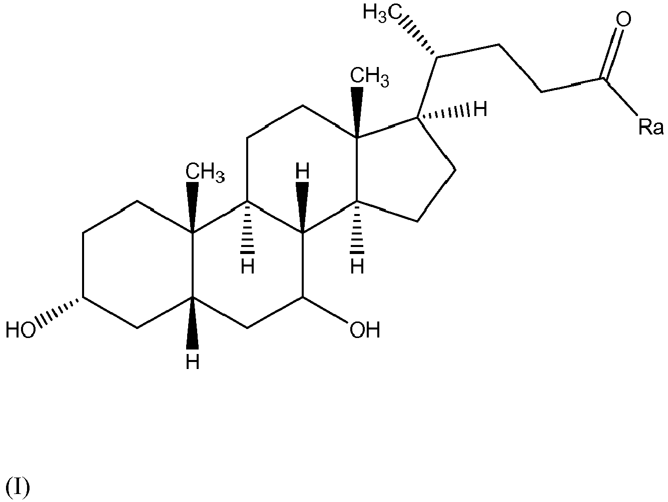 wherein Ra is -NR-CH-R₁-R₂ or -OH, wherein R is -H or C₁-C₄ alkyl; R₁ is -CH₂-SO₃R₃ and R₂ is -H; or R₁ is -COOH and R₂ is -CH₂-CH₂-CONH₂, -CH₂-CONH₂, -CH₂-CH₂-SCH₃ or -CH₂-S-CH₂-COOH; and R₃ is -H or the radical of a basic amino acid, or a pharmaceutically acceptable salt, stereoisomer, solvate or prodrug thereof, or a mixture thereof for use in the prevention and/or treatment of a disease caused by loss of function of ABCD1 gene, a disease caused by accumulation of very long fatty acids in organs and plasma or of a peroxisomal disorder.

Alternatively, the invention relates to a method for preventing and/or treating a disease caused by loss of function of ABCD1 gene, a disease caused by accumulation of very long fatty acids in organs and plasma or a peroxisomal disorder which comprises administering to a subject in need thereof a therapeutically effective amount of a compound of formula I 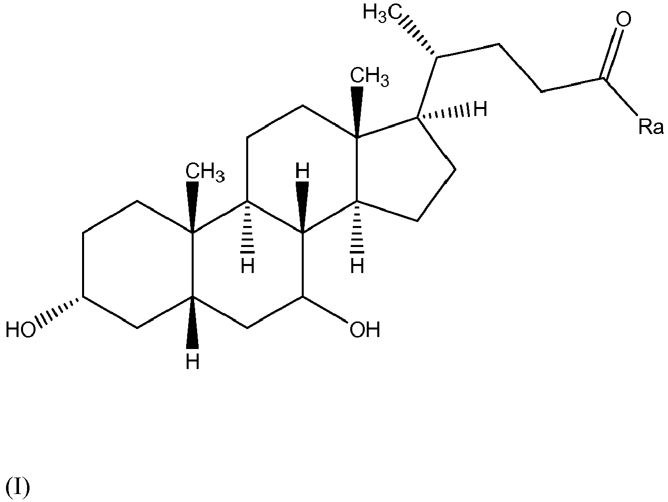 wherein Ra is -NR-CH-R₁-R₂ or -OH, wherein R is -H or C₁-C₄ alkyl; R₁ is -CH₂-SO₃R₃ and R₂ is -H; or R₁ is -COOH and R₂ is -CH₂-CH₂-CONH₂, -CH₂-CONH₂, -CH₂-CH₂-SCH₃ or -CH₂-S-CH₂-COOH; and R₃ is -H or the radical of a basic amino acid, or a pharmaceutically acceptable salt, stereoisomer, solvate or prodrug thereof, or a mixture thereof

Alternatively drafted, the invention relates to the use of a compound of formula I 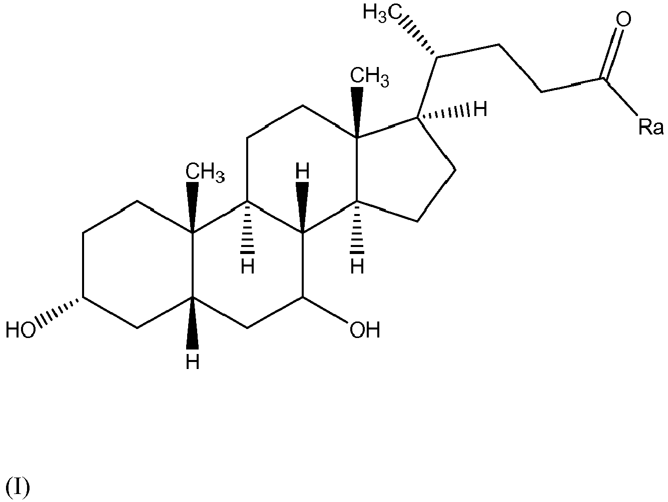 wherein Ra is -NR-CH-R₁-R₂ or -OH, wherein R is -H or C₁-C₄ alkyl; R₁ is -CH₂-SO₃R₃ and R₂ is -H; or R₁ is -COOH and R₂ is -CH₂-CH₂-CONH₂, -CH₂-CONH₂, -CH₂-CH₂-SCH₃ or -CH₂-S-CH₂-COOH; and R₃ is -H or the radical of a basic amino acid, or a pharmaceutically acceptable salt, stereoisomer, solvate or prodrug thereof, or a mixture thereof for the preparation of a medicament for the prevention and/or treatment of a disease caused by loss of function of ABCD1 gene, a disease caused by accumulation of very long fatty acids in organs and plasma or a peroxisomal disorder.

In another aspect, the invention relates to a combination of the invention as previously described for the prevention and/or treatment of a disease caused by loss of function of ABCD1 gene, a disease caused by accumulation of very long fatty acids in organs and plasma or a peroxisomal disorder.

Alternatively, the invention relates to a method for preventing and/or treating a disease caused by loss of function of ABCD1 gene, a disease caused by accumulation of very long fatty acids in organs and plasma or a peroxisomal disorder which comprises administering to a subject in need thereof a therapeutically effective amount of a combination of the invention as previously described.

Alternatively, the invention relates to a combination of the invention as previously described for the preparation of a medicament for the prevention and/or treatment of a disease caused by loss of function of ABCD1 gene, a disease caused by accumulation of very long fatty acids in organs and plasma or a peroxisomal disorder.

All the terms and embodiments previously described in relation to the combination of the invention are equally applicable to the medical uses described herein.

The term "prevention", "preventing" or "prevent", as used herein, relates to the administration of a compound of formula (I) or a combination comprising said compound according to the invention or of a medicament comprising said compound or combination to a subject who has not been diagnosed as possibly having a disease caused by loss of function of ABCD1 gene, a disease caused by accumulation of very long fatty acids in organs and plasma or of a peroxisomal disorder at the time of administration, but who would normally be expected to develop said disease or be at increased risk for said disease. The prevention intends to avoid the appearance of said disease. The prevention may be complete (e.g. the total absence of a disease). The prevention may also be partial, such that for example the occurrence of a disease in a subject is less than that which would have occurred without the administration of the inhibitor of the present invention. Prevention also refers to reduced susceptibility to a clinical condition.

The term "treatment", as used herein, relates to the administration of compound of formula (I) or a combination comprising said compound according to the invention or of a medicament comprising said compound or combination to a subject suffering from a disease caused by loss of function of ABCD1 gene, a disease caused by accumulation of very long fatty acids in organs and plasma or of a peroxisomal disorder including the administration in an initial or early stage of a disease, wherein the object is to prevent or slow down (lessen) an undesired physiological change or disorder. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. Treatment also means prolonging survival as compared to expected survival if not receiving the treatment.

The term "subject" as used herein, relates to any subject, particularly a mammalian subject, for whom therapy is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human of any race and sex.

"Disease caused by loss of function of ABCD1 gene", as used herein relates to any disease cause by point mutations or deletion in the ALD (ABCD1) gene that inactivate the ALD protein (ALDP). The loss of function of ALDP led to pathomechanisms of demyelination, inflammatory process, axonal degeneration or adrenal insufficiency. Point mutation or single base modification, is a type of mutation that causes a single nucleotide base substitution, insertion, or deletion of the genetic material, DNA or RNA. Deletion is a mutation (a genetic aberration) in which a part of a chromosome or a sequence of DNA is lost during DNA replication. Any number of nucleotides can be deleted, from a single base to an entire piece of chromosome.

"ABCD1 gene", also known as ATP binding cassette subfamily D member 1, or adrenoleukodystrophy protein (ALDP) codes the protein ABCD1 member of the ALD subfamily, which is involved in peroxisomal import of fatty acids and/or fatty acyl-CoAs in the organelle. ABCD1 is a half ABC-transporter, forming predominantly homodimers, but may also form heterodimers with other peroxisomal ABC-transporters. The full sequence of ABCD1 gene in humans has accession number Gene ID: 215 (3 July 2016)

"Disease caused by accumulation of very long fatty acids in organs and plasma", as used herein relates to a disease caused by accumulation of VLCFA, very long fatty acids with aliphatic tails longer than 22 carbons. Unlike most fatty acids, VLCFAs are too long to be metabolized in the mitochondria, and must be metabolized in peroxisomes. VLCFA accumulation may be measured using GC-MS (gas chromatography/mass spectrometry) method.

"Peroxisomal disorder", as used herein represents a class of medical conditions caused by defects in peroxisome functions. Illustrative non-limitative examples of peroxisomal disorder include the Zellweger syndrome spectrum (PBD-ZSD) and Infantile Refsum Disease, Neonatal Adrenoleukodystrophy, Hyperpipecolic acidaemia, Rhizomelic chondrodysplasia punctata type I; a disease caused byAcyl-CoA oxidase deficiency, a disease caused by D-bifunctional protein deficiency; a disease caused by 2-methylacylCoA racemase deficiency; Rhyzomelic chondrodysplasia punctata type 2 (DHAPAT deficiency); Rhyzomelic chondrodysplasia punctata type 3 (alkylDHAP synthase deficiency), Refsum Disease, Hyperoxaluria Type I; Glutaric academia type 3; a disease caused by Mevalonate kinase deficiency, Acatalasaemia, and Mulibrey nanism.

In a preferred embodiment, the disease is X-ALD.

"X-ALD" or X-linked adrenoleukodystrophy, ALD, X-ALD, Siemerling-Creutzfeldt disease or bronze Schilder disease that is linked on the X chromosome, relates to a metabolic genetic disorder of the central nervous system characterized by axonopathy in the spinal cords, progressive demyelination in the brain, and adrenal insufficiency. The disease is caused by loss of function of the ABCD1 gene, a peroxisomal ATP-binding cassette transporter, resulting in the accumulation of very long-chain fatty acids (VCLFA) in organs and plasma. These VCLFA build up in the brain. They damage the myelin that surrounds nerves. This can cause seizures and hyperactivity. It can also cause problems with speaking, listening and understanding verbal instructions.

In a more preferred embodiment the X-ALD shows AMN phenotype.

"AMN phenotype", as used herein relates to Adrenomyeloneuropathy comprising progressive neuropathy, paraparesis and approximately 40% of the patients progress to cerebral involvement

The present invention covers any combination of compounds and diseases.

For use according to the invention, the compound or the combination of the invention is present in a pharmaceutical composition together with a pharmaceutical acceptable excipient.

"Pharmaceutical composition" as used herein, relates to compositions and molecular entities that are physiologically tolerable and do not typically produce an allergic reaction or a similar unfavorable reaction as gastric disorders, dizziness and suchlike, when administered to a human or animal. Preferably, the term "pharmaceutically acceptable" means it is approved by a regulatory agency of a state or federal government or is included in the U.S. Pharmacopoeia or other generally recognized pharmacopoeia for use in animals, and more particularly in humans.

The term "excipient" refers to a vehicle, diluent or adjuvant that is administered with the active ingredient. Such pharmaceutical excipients can be sterile liquids, such as water and oils, including those of petroleum, animal, vegetable or synthetic origin, such as peanut oil, soybean oil, mineral oil, sesame oil and similars. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005; or "Handbook of Pharmaceutical Excipients", Rowe C. R.; Paul J. S.; Marian E. Q., sixth Edition

Suitable pharmaceutically acceptable vehicles include, for example, water, salt solutions, alcohol, vegetable oils, polyethylene glycols, gelatin, lactose, amylose, magnesium stearate, talc, surfactants, silicic acid, viscous paraffin, perfume oil, monoglycerides and diglycerides of fatty acids, fatty acid esters petroetrals, hydroxymethyl cellulose, polyvinylpyrrolidone and similars.

The pharmaceutical compositions containing the compound of formula (I) as defined above, or a pharmaceutically acceptable salt, stereoisomer or solvate thereof according to the invention can occur at any pharmaceutical form of administration considered appropriate for the selected administration route, for example, by systemic (e.g intravenous, subcutaneous, intramuscular injection), oral, parenteral or topical administration, for which it will include the pharmaceutically acceptable excipients necessary for formulation of the desired method of administration. Additionally, it is also possible to administer the composition comprising the compound of formula (I) as defined above, or a pharmaceutically acceptable salt, stereoisomer or solvate thereof of the invention intranasally or sublingually which allows systemic administration by a non-aggressive mode of administration. Also, intraventricular administration may be adequate. A preferred route of delivery is oral.

Those skilled in the art are familiar with the principles and procedures discussed in widely known.

Where necessary, the compound of formula (I) as defined above, the pharmaceutically acceptable salt, stereoisomer or solvate thereof or the combination of the invention is comprised in a composition also including a solubilizing agent and a local anesthetic to ameliorate any pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the composition is to be administered by infusion, it can be dispensed with an infusion bottle containing sterile pharmaceutical grade water or saline. Where the composition is administered by injection, an ampule of sterile water for injection or saline can be provided so that the ingredients may be mixed prior to administration.

In cases other than intravenous administration, the composition can contain minor amounts of wetting or emulsifying agents, or pH buffering agents. The composition can be a liquid solution, suspension, emulsion, gel, polymer, or sustained release formulation. The composition can be formulated with traditional binders and carriers, as would be known in the art. Formulations can include standard carriers such as pharmaceutical grades of mannitol, lactose, starch, magnesium stearate, sodium saccharide, cellulose, magnesium carbonate, etc., inert carriers having well established functionality in the manufacture of pharmaceuticals. Various delivery systems are known and can be used to administer a therapeutic of the present invention including encapsulation in liposomes, microparticles, microcapsules and the like.

Solid dosage forms for oral administration may include conventional capsules, sustained release capsules, conventional tablets, sustained-release tablets, chewable tablets, sublingual tablets, effervescent tablets, pills, suspensions, powders, granules and gels. At these solid dosage forms, the active compounds can be mixed with at least one inert excipient such as sucrose, lactose or starch. Such dosage forms can also comprise, as in normal practice, additional substances other than inert diluents, e.g. lubricating agents such as magnesium stearate. In the case of capsules, tablets, effervescent tablets and pills, the dosage forms may also comprise buffering agents. Tablets and pills can be prepared with enteric coatings.

Liquid dosage forms for oral administration may include emulsions, solutions, suspensions, syrups and elixirs pharmaceutically acceptable containing inert diluents commonly used in the technique, such as water. Those compositions may also comprise adjuvants such as wetting agents, emulsifying and suspending agents, and sweetening agents, flavoring and perfuming agents.

Injectable preparations, for example, aqueous or oleaginous suspensions, sterile injectable may be formulated according with the technique known using suitable dispersing agents, wetting agents and/or suspending agents. Among the acceptable vehicles and solvents that can be used are water, Ringer's solution and isotonic sodium chloride solution. Sterile oils are also conventionally used as solvents or suspending media.

For topical administration, compounds and combinations of the invention can be formulated as creams, gels, lotions, liquids, pomades, spray solutions, dispersions, solid bars, emulsions, microemulsions and similars which may be formulated according to conventional methods that use suitable excipients, such as, for example, emulsifiers, surfactants, thickening agents, coloring agents and combinations of two or more thereof.

Additionally, the compounds of formula (I) as defined above, the pharmaceutically acceptable salt, stereoisomer or solvate thereof or the combinations of the invention may be administered in the form of transdermal patches or iontophoresis devices. In one embodiment, the compounds or combinations of the invention are administered as a transdermal patch, for example, in the form of sustained-release transdermal patch. Suitable transdermal patches are known in the art.

Several drug delivery systems are known and can be used to administer the agents or compositions of the invention, including, for example, encapsulation in liposomes, microbubbles, emulsions, microparticles, microcapsules and similars. The required dosage can be administered as a single unit or in a sustained release form.

Sustainable-release forms and appropriate materials and methods for their preparation are described in, for example, "Modified-Release Drug Delivery Technology", Rathbone, M. J. Hadgraft, J. and Roberts, M. S. (eds.), Marcel Dekker, Inc., New York (2002), "Handbook of Pharmaceutical Controlled Release Technology", Wise, D. L. (ed.), Marcel Dekker, Inc. New York, (2000). In one embodiment of the invention, the orally administrable form of a compound or combination according to the invention is in a sustained release form further comprises at least one coating or matrix. The coating or sustained release matrix include, without limitation, natural polymers, semisynthetic or synthetic water-insoluble, modified, waxes, fats, fatty alcohols, fatty acids, natural semisynthetic or synthetic plasticizers, or a combination of two or more of the them.

Enteric coatings may be applied using conventional processes known to experts in the art, as described in, for example, Johnson, J. L., "Pharmaceutical tablet coating", Coatings Technology Handbook (Second Edition), Satas, D. and Tracton, A. A. (eds), Marcel Dekker, Inc. New York, (2001), Carstensen, T., "Coating Tablets in Advanced Pharmaceutical Solids", Swarbrick, J. (ed.), Marcel Dekker, Inc. New York (2001), 455-468.

In a preferred embodiment, the compound or combination of the invention is administered via oral route.

For use in the prevention and/or treatment according to the invention, the compound of formula (I) or a pharmaceutically acceptable salt, solvate or isomer thereof, the combination of the invention or the pharmaceutical composition of the invention is present in an effective amount.

The term "effective" amount or a "therapeutically effective amount" of a drug or pharmacologically active agent is meant a nontoxic but sufficient amount of the drug or agent to provide the desired effect. In the combination therapy of the present invention, an "effective amount" of one component of the combination is the amount of that compound that is effective to provide the desired effect when used in combination with the other components of the combination.

Even though individual needs vary, determination of optimal ranges for effective amounts of the agent of the invention belongs to the common experience of those experts in the art. In general, the dosage needed to provide an effective amount of such compound, which can be adjusted by one expert in the art will vary depending on age, health, fitness, sex, diet, weight, frequency of treatment and the nature and extent of impairment or illness, medical condition of the patient, route of administration, pharmacological considerations such as activity, efficacy, pharmacokinetic and toxicology profile of the particular compound used, if using a system drug delivery, and if the compound is administered as part of a combination of drugs.

The effective quantity of the compound of the invention can vary within a wide range and, in general, will vary depending on the particular circumstances of application, duration of the exposure and other considerations. In a particular embodiment, the dose ranges between 0.05 mg/kg and 50 mg/kg, more preferably between 1 mg/kg and 20 mg/kg. In a more preferred embodiment, the dose is between 1 and 3 g per day, more preferably 2 g per day.

In a preferred embodiment the effective amount is between about 0.005 % and about 0.04 % weight, between about 0.0075 % weight and about 0.0375 % weight, between about 0.001 % weight and about 0.035 % weight, between about 0.00125 % weight and about 0.0325 % weight, between about 0.0015 % weight and about 0.0325 % weight, between about 0.00175 % weight and about 0.03 % weight, and more preferably between about 0.0018 % weight and about 0.032 % weight. In a particular embodiment, the effective amount is between about 0.005% and about 0.02% weight, preferably between about 0.005% weight and about 0.015% weight, more preferably between about 0.005% weight and about 0.01% weight. In some embodiments the effective amount is about 0.001 % weight, about 0.002 % weight, about 0.003 % weight or about 0.004 % weight. The percentages (% w/w) are expressed as weight of the compound of formula (I) or a pharmaceutically acceptable salt, solvate or isomer thereof by the total weight of the composition comprising the compound.

In another embodiment the effective amount is expressed in µg/mL or µg/g (µg of the compound of formula (I) or a pharmaceutically acceptable salt, solvate or isomer thereof by mL or g of the composition comprising the compound), therefore effective amount is about 75 and about 375 µg/mL (or µg/g), between about 100 and about 350 µg/mL (or µg/g), between about 125 and about 325 µg/mL (or µg/g), between about 150 and about 325 µg/mL (or µg/g), between about 175 and about 300 µg/mL (or µg/g), and more preferably between about 180 and about 320 µg/mL (or µg/g). In a particular embodiment, the effective amount is between about 50 and about 200 µg/mL (or µg/g), preferably between 50 and about 150 µg/mL (or µg/g), more preferably between about 50 and about 100 µg/mL (or µg/g). In some embodiments the effective amount is about 100 µg/mL (or µg/g), about 200 µg/mL (or µg/g), about 300 µg/mL (or µg/g) or about 400 µg/mL (or µg/g).

When the compound of formula (I) or a salt, solvate or isomer thereof as defined herein is present on a surface, it is preferably in an effective amount of between about 1 and about 200 µg/cm², preferably between about 1 and about 100 µg/cm², preferably between about 1 and about 50 µg/cm², more preferably between about 5 and about 300 µg/cm².

All the terms and embodiments previously described are equally applicable to this aspect of the invention.

The invention will be described by way of the following examples which are to be considered as merely illustrative and not limitative of the scope of the invention.

### EXAMPLES

### MATERIAL AND METHODS

### X-ALD mice

The generation and genotyping of *Abcd1* null mice have been previously described (Pujol A. et al., Hum Mol Genet 2002; 11:499-505, Lu JF. et al., Proc.Natl.Acad.Sci.U.S.A 1997; 94:9366-9371.). Mice used for experiments were of a pure C57BL/6J background. *Abcd1* null and WT mice were separated into control and treated groups. Treated groups were fed with a mixture of antioxidants that included 1000 iu/kg Vitamin E and 0.5% α-lipoic acid in the diet and 1% N-acetyl-cysteine (NAC) in water. Daily feedings were initiated at 8 months of age and were continued for 4 months until sacrifice.

The bile acid tauroursodeoxycholic acid TUDCA (Prodotti Chimici e Alimentari S.p.A., Italy) was mixed into AIN-76A chow (Dyets) at 0.4% w/w. Male mice were randomly assigned to one of the following groups: wild-type mice (*n*=10) that received normal AIN-76A chow, *Abcd1*^{*-*/*-*} mice (*n*=10) that received normal AIN-76A chow and *Abcd1^{-l-}* mice (*n*=10) that received AIN-76A chow containing TUDCA. The animals were treated for 3 months starting at 9 months of age to assess its effect on the progression of the biochemical signs of X-linked adrenoleukodystrophy. The double knockout mice and their control littermates were randomly assigned to one of the following groups: wild-type mice (*n*=10) that received normal AIN-76A chow; double knockout mice (*n* =10) that received normal AIN-76A chow; and double knockout mice (*n* =10) that received AIN-76A chow containing TUDCA. The animals were treated for 4 months starting at 13 months of age to coincide with the onset of clinical signs. TUDCA had no effect on weight or food intake under either treatment protocol.

For behavioral testing, assessment of hindlimb clasping was done by suspending mice from their tails until it reached a vertical position, but allowing the mice to grab the grill of the lid of the cage with the forelimbs, adapted from Dumser et al. cited supra. Hindlimb reflex was analysed for 10s in three consecutive trials separated by 5 min rest. The treadmill and bar cross experiments were performed exactly as previously described (Lopez-Erauskin J. et al., Ann Neurol 2011; 70:84-92,, Morato L. et al., Brain 2013; 136:2432-43.) Mock- and drug-treated mice in each litter were simultaneously tested using the clasping, the treadmill and bar cross for both genotype and treatment in a blind study.

All methods employed in this study were in accordance with the Guide for the Care and Use of Laboratory Animals published by the US National Institutes of Health (NIH publication No. 85-23, revised 1996) and the ethical committees of IDIBELL and the Generalitat de Catalunya.

### Immunoblot analysis

Tissues were lysed in ice-cold RIPA buffer (50 mM Tris-HCl, pH 8, 12 mM deoxycholic acid, 150 mM NaCl and 1% NP40, supplemented with Complete Protease Inhibitor Cocktail, Roche) using a Teflon-on-glass homogenizer and then centrifuged at 1500 x g for 10 min at 4°C. The protein concentration was determined using a BCA protein assay kit (Thermo Fisher Scientific, Inc.). Samples were boiled for 5 min in Laemmli's buffer and run on SDS-polyacrylamide gels. After electrophoresis, proteins were transferred to nitrocellulose membranes (Protran blotting membrane, Whatman GmbH) for blotting with appropriate antibodies. Proteins were visualized with an enhanced chemiluminescence western blot detection system (GE Healthcare BioSciences AB), followed by exposure to CL-XPosure Film (Thermo Scientific).

### Immunohistochemistry

Spinal cords were harvested from 3 and 12-month-old wild type and *Abcd1⁻* mice, after perfusion with 4% paraformaldehyde (Lopez-Erauskin J. et al., 2011 cited supra, Ferrer I. et al., Hum Mol Genet 2005; 14:3565-77). Spinal cords were embedded in paraffin and serial sections (4-µm thick) were cut in a transversal or longitudinal (1-cm long) plane. Immunohistochemistry was carried out with the avidin-biotin peroxidase method and at least five sections of the spinal cord were analysed per animal and per stain. The sections were processed for immunohistochemistry to GRP78, PDi, Iba1, GFAP, synaptophysin and APP. The results were expressed as the mean ± standard deviation.

For immunofluorescence, confocal images were acquired using a Leica TCS SL laser scanning confocal spectral microscope (Leica Microsystems Heidelberg GmbH, Mannheim, Germany) and images were analyzed with ImageJ 1.39 u (NIH, USA).

### Statistical analysis

Statistical significance (P < 0.05) was assessed using the Student's *t*-test whenever two groups were compared. When analyzing multiple groups, ANOVA and Tukey's *post-hoc* test were used to determine statistical significance. Data are presented as mean + SD and p < 0.05 was considered significant (*, p < 0.05; **, p < 0.01; ***, p < 0.001).

### Example 1. Antioxidant treatment prevents ER stress and UPR induction in Abcd1 null mice

The inventors previously showed that a combination of vitamin E, α-lipoic acid and N-acetyl cysteine (NAC) efficiently reduced ROS production *in vitro,* reversed oxidative damage to proteins and DNA in spinal cords, and arrested axonal degeneration and disability in X-ALD mice (Lopez-Erauskin J. et al, 2011 cited supra).

To determine whether ER stress is regulated by redox imbalance *in vivo,* the inventors treated with antioxidants during 4 months the presymptomatic Abcd1 null mice at 8 months of age. Next, they assessed by Western Blots the presence of ER stress and UPR responses.

Mice treated with antioxidants showed a downregulation of PERK and eif2α phosphorylation, cleaved-ATF6, ATF4 and CHOP (Figure 1A). Moreover, the treatment prevented the downregulation of GRP94 and GRP78 and the induction of PDI (Figure 1B) at the late time point studied, suggesting that oxidative stress causes UPR dysfunction during X-ALD disease progression. Thus, these data i) confirm that oxidative stress induces ER stress and UPR activation in X-ALD mice, and ii) suggest failure in the synthesis of UPR-associated chaperones, or a blunted UPR response, in aged X-ALD mice.

### Example 2. TUDCA reduces the ER stress in Abcd1 null mice

The mouse model of the disease, the Abcd1 null mouse, develops a late onset axonopathy that resembles the most frequent X-linked adrenoleukodystrophy phenotype, AMN. WT and *Abcd1* null mice were fed with a diet supplemented with either 0.4 % (wt/wt) TUDCA or vehicle for 3 months. The dose and duration of TUDCA, whose pharmacokinetics has been extensively studied in rodents Crosignani A. et al., Clin Pharmacokinet 1996; 30:333-58], has been chosen based on pilot studies using APP/PS1 mice and other animal studies (Castro RE. et al., Am J Physiol Gastrointest Liver Physiol; 299:G887-97., Nunes AF. et al., Mol Neurobiol. 2012 Jun;45(3):440-54). Then, the inventors determined the protein levels of ER stress marker dysregulated in the *Abcd1* null mice. Indeed, ATF6, P-PERK, P-eif2α/eif2α, ATF4 and CHOP levels were normalized in *Abcd1* null mice exposed to TUDCA (Figure 1C). Moreover, TUDCA also normalized the levels of the ER chaperones GRP78 and GRP94 and prevented PDI upregulation (Figure 1D).

### Example 3. TUDCA prevents axonal degeneration in Abcd1⁻/Abcd2^{-/-} mice

To ascertain potential therapeutic effects of drugs of interest, the inventors used double null *Abcd1⁻*/*Abcd2*^{*-*/*-*} mice. The *Abcd2* gene is the closest homolog to *Abcd1* and exhibits overlapping biochemical and physiological functions with *Abcd1.* Overexpression of Abcd2 is sufficient to compensate for the neurological phenotype caused by *Abcd1* loss *in vivo.* These double mutant mice exhibited a more pronounced and earlier-onset axonal degenerative phenotype, manifesting at around 12 months of age instead of 20 months in the *Abcd1* null mice, what makes them better suitable for therapeutic assays (Launay N,et al., Acta Neuropathol 2015; 129:399-415.). The neuropathological phenotype of *Abcd1⁻*/*Abcd2*^{*-*/*-*} mice is characterized by the following: (1) microgliosis and astrocytosis, as demonstrated by Iba-1 and glial fibrillary acidic protein (GFAP) staining, respectively; (2) axonal damage, as demonstrated by amyloid precursor protein (APP) and synaptophysin accumulation in axonal swellings; and (3) scattered myelin debris, as demonstrated by Sudan black staining (Figure 2A). Interestingly, axonal damage markers and the numbers of reactive astrocytes and reactive microglia were strikingly reduced to control levels with TUDCA treatment (Figures 2A, 2B).

### Example 4. TUDCA halts locomotor deficits progression in Abcd1⁻/Abcd2^{-/-} mice

Locomotor deficits in *Abcd1* /*Abcd2*^{*-*/*-*} mice were evaluated using clasping, treadmill and bar cross experiments after TUDCA treatment as described (Dumser M- et al., 2007 cited supra, Launay N. et al., 2015 cited supra). In the clasping test, the inventors found that the *Abcd1* /*Abcd2*^{*-*/}*⁻ mice* presented an average score of 1, corresponding to hind limbs partially retracted towards the abdomen during 100% of the time, in contrast to wild type mice who presented an average score of 2 (Figure 3A). Interestingly, TUDCA treatment improved noticeably the median clasping score which changed from 1 to 1.75, equivalent to alternating clasping and more frequent flexions of hind limbs. In the treadmill experiment, the double-knockout mice exhibited longer shock times and more shocks compared with wild-type mice, which indicate locomotor disability before treatment. After 3 months of treatment, this ratio was indistinguishable from wild types, indicating that TUDCA treatment had stopped the progression of locomotor deficits in X-linked adrenoleukodystrophy mice (Figure 3B). Finally, in the bar cross experiments, double-knockout mutants often failed to maintain their balance and displayed a greater tendency to slip off the bar as well as longer latencies for reaching the platform at the opposite end of the bar (Morato L. et al., Brain 2013; 136:2432-43, Morato L. et al., Cell Death Differ 2015; 22:1742-53). The number of slips and time necessary to cross the bar were also normalized following TUDCA treatment (Figure 3C). Overall, these data indicate that TUDCA treatment arrests disease progression in *Abcd1⁻*/*Abcd2*^{*-*/*-*} mice.

## Claims

1. A compound of formula I 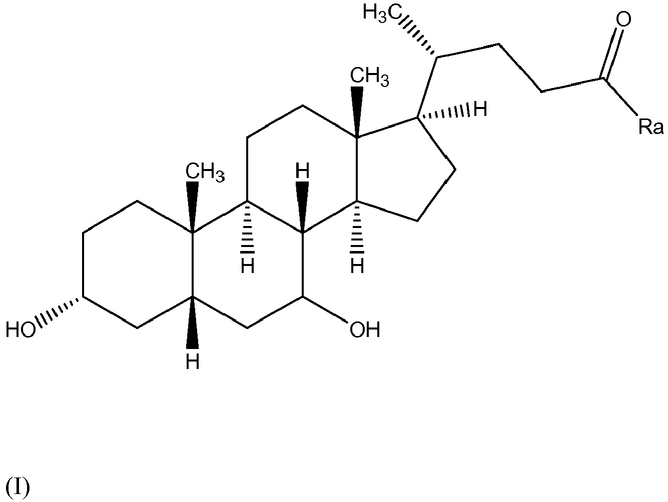 wherein Ra is -NR-CH-R₁-R₂ or -OH, wherein R is -H or C₁-C₄ alkyl; R₁ is -CH₂-SO₃R₃ and R₂ is -H; or R₁ is -COOH and R₂ is -CH₂-CH₂-CONH₂, -CH₂-CONH₂, -CH₂-CH₂-SCH₃ or -CH₂-S-CH₂-COOH; and R₃ is -H or the radical of a basic amino acid,
or a pharmaceutically acceptable salt, stereoisomer, solvate or prodrug thereof, or a mixture thereof for use in the prevention and/or treatment of a disease caused by loss of function of ABCD1 gene, a disease caused by accumulation of very long fatty acids in organs and plasma or of a peroxisomal disorder.

2. The compound for use according to claim 1 of formula 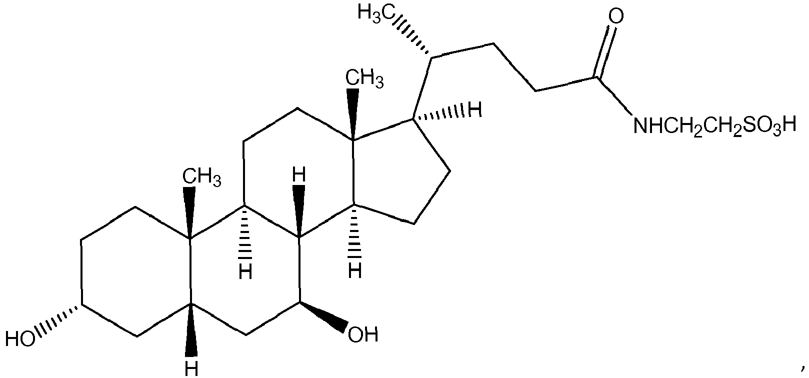 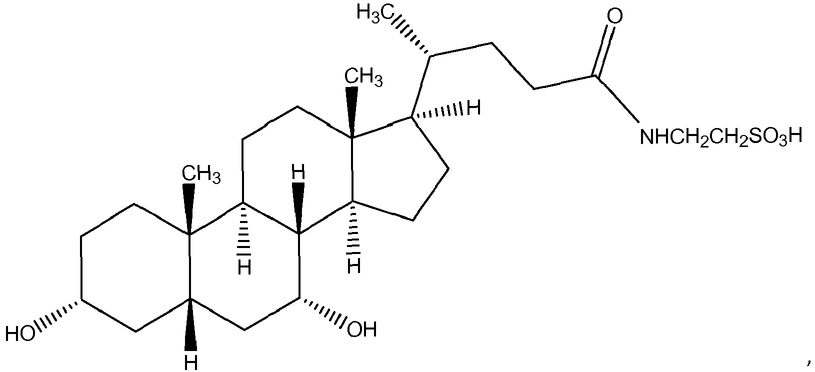 or

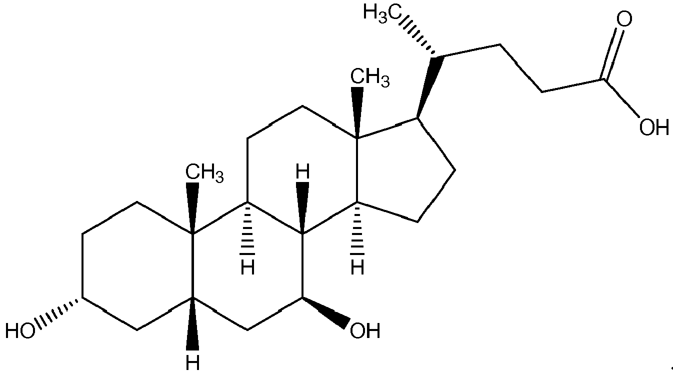

3. The compound for use according to any of claims 1-2 wherein the disease caused by accumulation of very long fatty acids in organs and plasma or loss of function of ABCD1 gene is X-ALD and/or , the peroxisomal disorder is selected from the group consisting of Zellweger syndrome, Infantile Refsum Disease, Neonatal Adrenoleukodystrophy, Hyperpipecolic acidaemia, Rhizomelic chondrodysplasia punctata type I; a disease caused by Acyl-CoA oxidase deficiency, a disease caused by D-bifunctional protein deficiency; a disease caused by 2-methylacylCoA racemase deficiency; Rhyzomelic chondrodysplasia punctata type 2; Rhyzomelic chondrodysplasia punctata type 3, Refsum Disease, Hyperoxaluria Type I; Glutaric academia type 3; a disease caused by Mevalonate kinase deficiency, Acatalasaemia, and Mulibrey nanism.

4. The compound for use according to any of claims 1-3 wherein the disease is X-ALD.

5. The compound for use according to claim 4, wherein the X-ALD shows AMN phenotype.

6. The compound for use according to any of claims 1-5 wherein the compound is administered orally.

7. A combination comprising (i) a compound of formula (I) 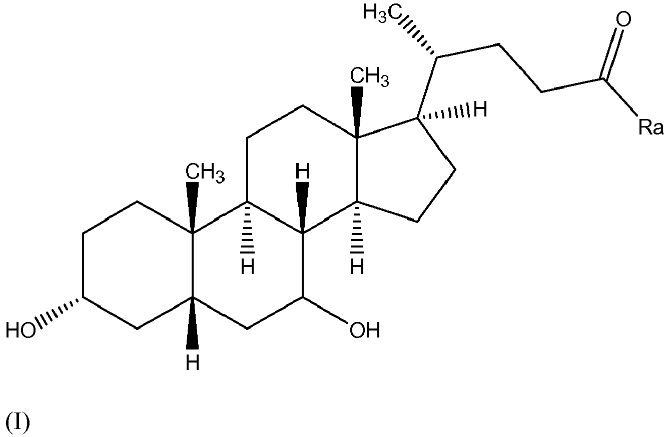 wherein Ra is -NR-CH-R₁-R₂ or -OH, wherein R is -H or C₁-C₄ alkyl; R₁ is -CH₂-SO₃R₃ and R₂ is -H; or R₁ is -COOH and R₂ is -CH₂-CH₂-CONH₂, -CH₂-CONH₂, -CH₂-CH₂-SCH₃ or -CH₂-S-CH₂-COOH; and R₃ is -H or the radical of a basic amino acid, or a pharmaceutically acceptable salt, stereoisomer, solvate or prodrug thereof, or a mixture thereof and (ii) an antioxidant, an activator of mitochondria biogenesis, an anti-inflammatory compound, an activator of autophagy, a peroxisomal biogenesis activator or a mixture thereof.

8. A combination according to claim 7 wherein the compound (i) is 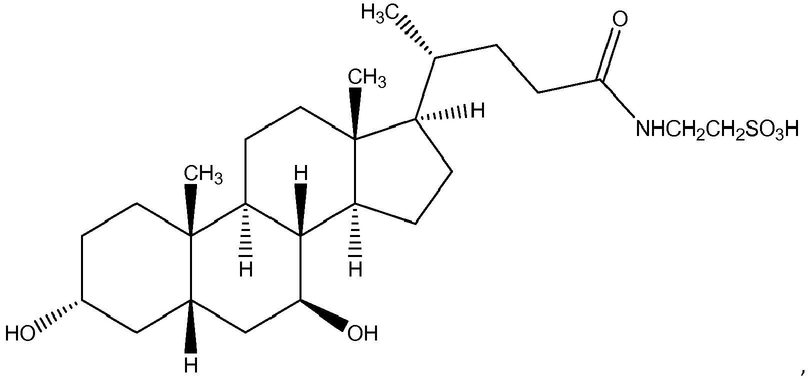 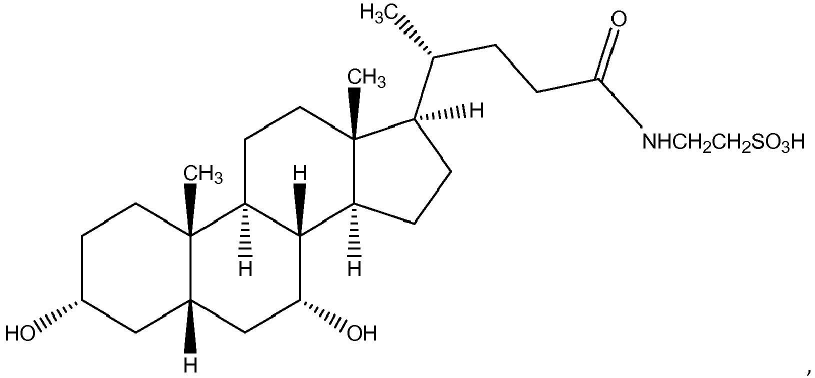 or

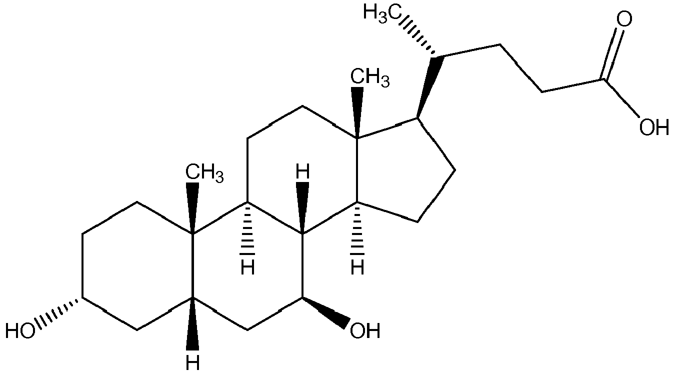

9. A combination according to claim any of claims 7 or 8 wherein the antioxidant is selected from the group consisting of Vitamin E, α-lipoic acid, N-acetyl-cysteine and combinations thereof.

10. A combination according to any of claims 7 to 9 wherein the activator of mitochondria biogenesis is selected from pioglitazone and derivatives and combinations thereof, the activator of autophagy is selected from temsirolimus, derivatives and combinations thereof and/or the antiinflammatory compound is selected from the group consisting of fingolimod, siponimod and combinations thereof and the peroxisomal biogenesis activator is sodium butyrate.

11. A combination according to any of claims 7 to 10 for use in the prevention and/or treatment of a disease caused by loss of function of ABCD1 gene, a disease caused by accumulation of very long fatty acids in organs and plasma or of a peroxisomal disorder.

12. The combination for use according to claim 11, wherein the disease caused by accumulation of very long fatty acids in organs and plasma is X-ALD or the peroxisomal disorder is selected from the group consisting of Zellweger syndrome, Infantile Refsum Disease, Neonatal Adrenoleukodystrophy, Hyperpipecolic acidaemia, Rhizomelic chondrodysplasia punctata type I; a disease caused byAcyl-CoA oxidase deficiency, a disease caused by D-bifunctional protein deficiency; a disease caused by 2-methylacylCoA racemase deficiency; Rhyzomelic chondrodysplasia punctata type 2 (DHAPAT deficiency); Rhyzomelic chondrodysplasia punctata type 3 (alkylDHAP synthase deficiency), Refsum Disease, Hyperoxaluria Type I; Glutaric academia type 3; a disease caused by Mevalonate kinase deficiency, Acatalasaemia, and Mulibrey nanism.

13. The combination for use according to claim 12, wherein the disease is X-ALD.

14. The combination for use according to claim 13, wherein the X-ALD shows AMN phenotype.

15. The combination for use according to any of claims 11 to 14 wherein the combination is administered orally.
